# EUROPEAN PATENT SPECIFICATION

(11) **EP 1 957 028 B1**
(45) Date of publication and mention of the grant of the patent: **07.06.2017**
(21) Application number: 06809789.8
(22) Date of filing: 26.10.2006
(51) Int. Cl.: A61J 1/14, A61J 1/10, A61J 1/20, A61M 39/04, A61M 39/26, A61M 5/162

(54) **NEEDLELESS ADDITIVE CONTROL VALVE**
NADELLOSES ZUSATZSTOFF-KONTROLLVENTIL
VANNE SANS AIGUILLE POUR CONTRÔLE D ADDITIF

(30) Priority: 30.10.2005 IL 17166205
(43) Date of publication of application: 20.08.2008
(73) Proprietor: Medimop Medical Projects Ltd., 43665 Ra'anana (IL)
(72) Inventor: ZINGER, Freddy, 43611 Ra'anana (IL); DENENBURG, Igor, 2107 Beberi, Riga District (LV)
(74) Representative: South, Nicholas Geoffrey
(86) International application number: PCT/IL2006/001228
(87) International publication number: WO 2007/052252

(56) References cited:
- WO-A-03/079956
- US-A- 5 645 538
- US-A1- 2003 191 445
- US-A1- 2003 199 847

## Description

### Field of the Invention

The invention pertains to additive control valves for use with fluid containers containing an infusion liquid and an intravenous (IV) or administration port for administrating the infusion liquid.

### Background of the Invention

Conventional infusion bags having an intravenous (IV) or administration port for interconnection with an infusion set, a drip chamber, and a spike, and a self-sealing additive port for enabling needle injection of a liquid additive. However, needle injection into an infusion bag's additive port can inadvertently mix scrapings from its additive port with its infusion contents. Moreover, some needles have shrouds to avoid needle sticks but which preclude insertion into an additive port thus limiting certain shrouded needles to be used with infusion bags having specific additive ports thereby complicating the administration of medication to patients.

US Patent No. 4,581,014 to Millerd et al. illustrates and describes a fluid infusion system enabling interruption of a primary infusion fluid to a patient to permit administration of a secondary infusion fluid followed by resumption of primary fluid flow. The fluid infusion system includes a selector valve (12) with an upstanding primary spike (36) for puncturing an IV port (25) of an infusion bag, a generally upstanding dual path secondary spike (44) for puncturing a vial's self-sealing rubber stopper, and an outlet fitting (54) for connection to the upper end of a drop chamber.

US Patent No. 5,647,845 to Haber et al. illustrates and describes a universal intravenous infusion system enabling mixing of a customized or generic fluid or powder medication with the fluid contents of an infusion bag so that the mixture can be administered according to the needs of a patient. The intravenous infusion system includes a fluid control valve (2) with an administration port cannula (14), a vial receiving and docking receptacle (20), and an IV drip chamber tube (18) with a fluid port (19) for mating to a conventional IV fluid line.

US 2003/0191445 A1 discloses a device and method for mixing medical fluids. The device has an inlet port, a self-sealing injection port, an outlet port for receiving an IV spike, a first duct between the injection port and the inlet port, and a second duct between the inlet port and the outlet port. The injection port is sealed by a fluid-proof membrane which can be penetrated by an injection needle.

US 2003/0199847 A1 discloses a fluid transfer device for use in an infusion system having a first end and a second end for coupling to an injection port of the infusion system. The device further includes at least a first member, a hollow needle attached to the first member, and a second member telescopically displaceable in relation to the first member, allowing the hollow needle to penetrate a flexible barrier member sealing the injection port thereby creating a fluid passage into the infusion system. The first end has a connecting portion for attachment to a drug bottle containing a fixed dose of a medical substance, and the second end has a flexible membrane able to be pressed against the flexible barrier member with a pressure sufficient to create a double-membrane sealing around the hollow needle.

WO 03/079956 A1 discloses a fluid transfer assembly, drug container, and method for enabling fluid transfer in an infusion system. The assembly includes a fluid container containing an infusion fluid, and a drug container. The fluid container has at least one inlet port for receiving a medical substance from the drug container. The assembly further includes at least one fluid barrier controlling fluid passage between the drug container and the fluid container. The inlet port exhibits a first luer-lock connector, and the drug container is sealed by a cap exhibiting a second luer-lock connector for attachment to the first luer-lock connector. The fluid barrier is designed and arranged to be ruptured by an external force to allow the fluid passage.

### Summary of the Invention

The present invention is directed towards a needleless additive control valve, as claimed in claim 1. The needleless additive control valve of the present invention affords a simple low cost device for use with conventional additive transfer devices with male connectors and enabling access to a fluid container by way of an IV infusion set, a drip chamber, a spike, and the like. The male connectors are preferably male Luer connectors of either the slip type or the lock type.

### Brief Description of the Drawings

In order to understand the invention and to see how it can be carried out in practice, preferred embodiments will now be described, by way of non-limiting examples only, with reference to the accompanying drawings in which similar parts are likewise numbered, and in which:
Fig. 1 is a pictorial view of an infusion bag, a needleless additive control valve, a syringe, a vial adapter, and a vial;
Fig. 2A is a front view of the needleless additive control valve with a self-sealing access valve; Fig. 2B is a front view of the self-sealing access valve;
Fig. 2B is a front view of the self-sealing access valve;
Fig. 3A is a cross section of the self-sealing access valve in its closed condition along line A-A in Figure 2A;
Fig. 3B is a cross section of the self-sealing access valve in its open condition along line A-A in Figure 2A;
Figs. 4A and 4B show the use of the needleless additive control valve with a syringe containing a liquid additive; and
Figs. 5A and 5B show the use of the needleless additive control valve with a vial adapter mounted on a vial containing a liquid additive.

### Detailed Description of Preferred Embodiments of the Present Invention

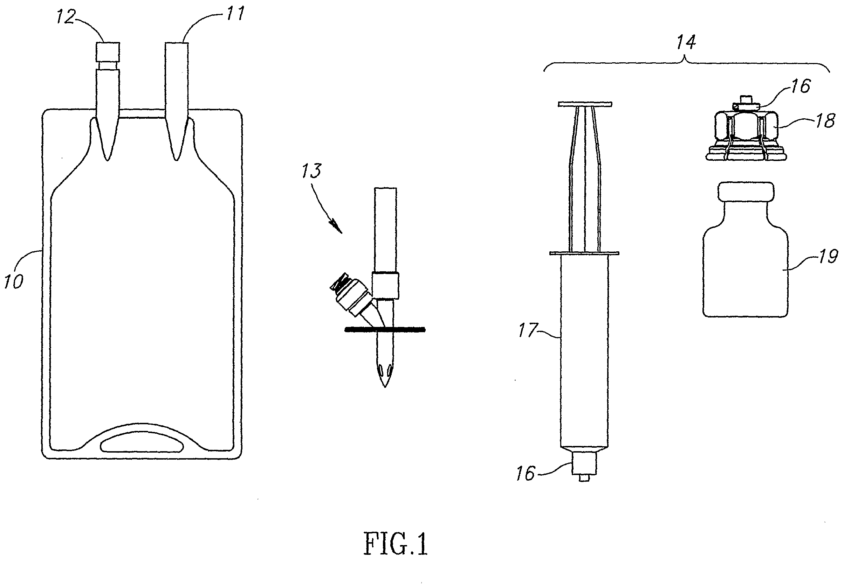

Figure 1 shows a conventional infusion bag 10 containing an infusion liquid and having an intravenous (IV) or administration port 11 and an additive port 12, a needleless additive control valve 13, and an additive transfer device 14 having a male Luer lock connector 16. The additive transfer device 14 can be constituted by a syringe 17, a vial adapter 18 for snap fit telescopic mounting onto a vial 19, and the like. Suitable vial adapters 18 with integrally formed puncturing members for puncturing a vial's rubber stopper are commercially available from Medimop Medical Projects Ltd., Ra'anana, Israel (**www.medimop.com**). The vial 19 can contain a liquid additive or a lyophilized powder drug requiring reconstitution with a diluent prior to administration.

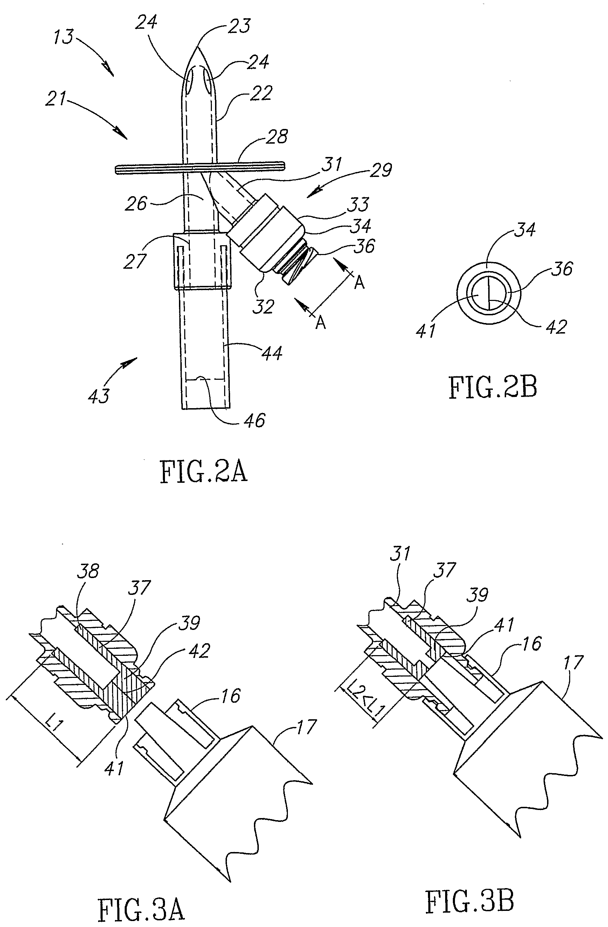

Figure 2 shows the needleless additive control valve 13 has a trifurcated connector body 21. The trifurcated connector body 21 includes an IV spike 22 for sealing insertion into an IV port 11. The IV spike 22 is formed from suitable rigid plastic material such as polycarbonate, and the like. The IV spike 22 includes a spiked end 23 with peripherally disposed apertures 24, a lumen 26, and an outlet aperture 27. The IV spike 22 is integrally formed as an injection molded monolithic structure with a circular flange 28 for restricting insertion into the IV port 11 and a normally closed (NC) needleless additive port 29 disposed toward the outlet aperture 27.

The needleless additive port 29 has a lumen 31 and is fitted with a self-sealing access valve 32 for screw threadingly receiving a male Luer lock connector 16. The access valve 32 is typically formed from rigid transparent plastic material such as polycarbonate, and the like. The access valve 32 has a stepped exterior surface 33 including an abutment surface 34 and a rim 36. The access valve 32 houses a silicone self-sealing valve member 37 with a tubular main portion 38 and a cylindrical needleless entry portion 39. The needleless entry portion 39 has an exposed entry surface 41 and a pre-formed slit 42 extending therealong. The valve member 37 has a natural length L1 and is so dimensioned that the entry surface 41 is flush with the rim 36 thereby enabling the entry surface 41 to be readily swabbed for sterilization purposes (see Figure 3A). A male Luer connector 16 screw threaded onto the access valve 32 advances until it abuts against the abutment surface 34. During its advancement, the male Luer connector 16 compresses the valve member 37 to a compressed length L2<L1 which parts the entry portion 39 along its pre-formed slit 42 for enabling fluid communication between an additive transfer device and the lumen 31 (see Figure 3B).

The trifurcated connector body 21 includes a substitute IV port 43 attached to the outlet aperture 27, and having a lumen 44 and a sealing membrane 46. The substitute IV port 43 is a conventional IV port made from suitable flexible plastic material, for example, PVC, and the like, for sealingly receiving an about 5mm to 6mm diameter IV spike (not shown).

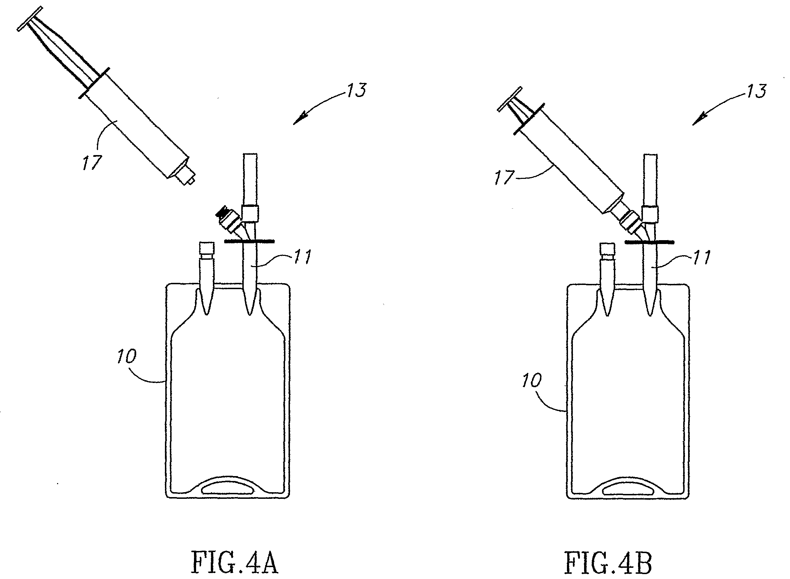

Figures 4A and 4B show the use of the needleless additive control valve 13 with an infusion bag 10 and a syringe 17. A syringe 17 can be pre-filled with a liquid additive. Alternatively, a syringe 17 can be filled with a liquid drug reconstituted from a lyophilized powder drug vial. A reconstituted liquid drug may have been reconstituted with liquid contents aspirated from an infusion bag. The syringe 17 can be employed for injecting a liquid additive into the infusion bag 10 prior to administration of infusion liquid to a patient. Alternatively, the syringe 17 can be employed for administrating a liquid additive to a patient during an infusion procedure.

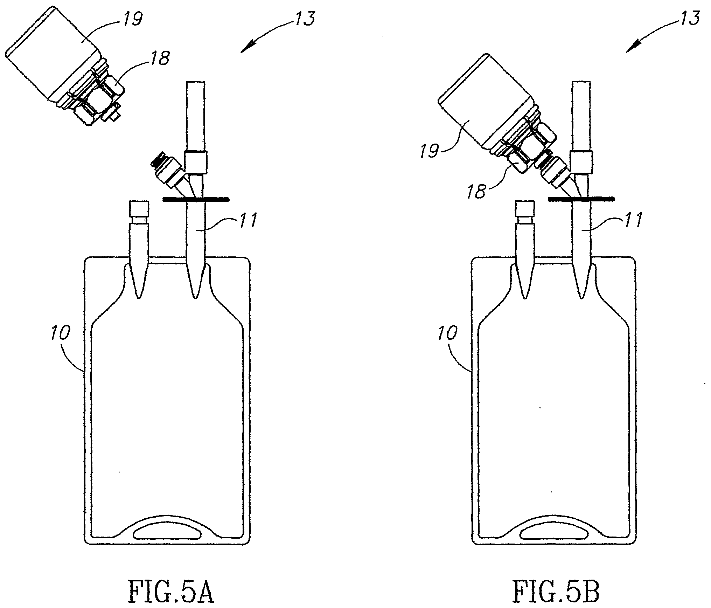

Figures 5A and 5B show the use of the needleless additive control valve 13 with an infusion bag 10 and a vial adapter 18 mounted on a vial 19. In the case that the vial 19 contains a liquid additive, the contents of the vial 19 can be mixed with an infusion liquid prior to administration of infusion liquid to a patient or administered to a patient during an infusion procedure. In the case that the vial 19 contains a lyophilized powder drug, the drug can be reconstituted by squeezing on the infusion bag 10 for forcing liquid from the infusion bag 10 into the vial 19. The reconstitution of the entire contents of a vial 19 may require several flushes with diluent.

While the invention has been described with respect to a limited number of embodiments, it will be appreciated that many variations, modifications, and other applications of the invention can be made within the scope of the appended claims. For example, the self-sealing access valve can be replaced by a manually operated stop cock, and the like.

## Claims

1. An additive control valve (13) for use with a fluid container (10) containing an infusion liquid and having an intravenous (IV) port (11) for administrating the infusion liquid, and an additive transfer device (14) containing a liquid additive and having a male connector (16) for administrating the liquid additive,
the additive control valve (13) comprising a trifurcated connector body (21) having an IV spike (22) for sealing insertion into the IV port (11), a substitute IV port (43) for sealingly receiving an IV spike, and a normally closed additive port (29) with a female connector for sealingly receiving the additive transfer device's male connector (16),
said IV spike (22) being in direct and continuous fluid communication with said substitute IV port (43), and said normally closed additive port (29) being in direct and continuous fluid communication with said IV spike whereby the additive transfer device's liquid additive is able to be mixed with the infusion liquid,
wherein said additive port (29) includes a self-sealing access valve (32) urged from a normally closed condition to an open condition on a sealing insertion of a male connector therein,
**characterised in that**
said additive port (29) is a needleless additive port (29) arranged to receive a needleless male connector of a needleless additive transfer device and includes an exposed needleless access surface (41) having a pre-formed slit for receiving the needleless male connector so as to urge the self-sealing access valve (32) from the normally closed condition to the open condition on a sealing insertion of the needleless male connector therein, the exposed needleless access surface (41) being flush with a distal rim (36) of the additive port (29) whereby the exposed needleless access surface (41) is capable of being sterilized for multiple sterile sealing insertions of the needleless male connector therethrough,
and said needleless additive port (29) is in direct and continuous fluid communication with said substitute IV port (43) such that said IV spike, said normally closed needleless additive port and said substitute IV port are in three way direct and continuous fluid communication, whereby the additive transfer device's liquid additive is alternatively able to be directly administered to a patient.

2. The valve according to claim 1 wherein a male connector is screw threadingly mounted on said needleless additive port.

3. The valve according to claim 1 or 2 wherein said IV spike (22) and said needleless additive port (29) are an injection molded plastic monolithic structure.

## Patentansprüche

1. Zusatzstoff-Steuerventil (13) zur Verwendung mit einem Flüssigkeitsbehältnis (10), das eine Infusionsflüssigkeit enthält und einen intravenösen Port (iv-Port) (11) zum Verabreichen der Infusionsflüssigkeit aufweist, und eine Zusatzstoff-Transfervorrichtung (14), die einen flüssigen Zusatzstoff enthält und einen männlichen Konnektor (16) zum Verabreichen des flüssigen Zusatzstoffs aufweist,
wobei das Zusatzstoff-Steuerventil (13) Folgendes umfasst: einen dreifach gegabelten Konnektorkörper (21) mit einem iv-Einstechdorn (22) zur abdichtenden Einbringung in den iv-Port (11), einem Ersatz-iv-Port (43) zur abdichtenden Aufnahme eines iv-Einstechdorns und einem normalerweise geschlossenen Zusatzstoff-Port (29) mit einem weiblichen Konnektor zur abdichtenden Aufnahme des männlichen Konnektors (16) der Zusatzstoff-Transfervorrichtung,
wobei sich der iv-Einstechdorn (22) in direkter und kontinuierlicher Fluidkommunikation mit dem Ersatz-iv-Port (43) befindet, und sich der normalerweise geschlossene Zusatzstoff-Port (29) in direkter und kontinuierlicher Fluidkommunikation mit dem iv-Einstechdorn befindet, wobei der flüssige Zusatzstoff der Zusatzstoff-Transfervorrichtung mit der Infusionsflüssigkeit gemischt werden kann,
wobei der Zusatzstoff-Port (29) ein selbstdichtendes Zugangsventil (32) einschließt, das nach einer abdichtenden Einbringung eines männlichen Konnektors dort hinein aus einem normalerweise geschlossenen Zustand in einen offenen Zustand gebracht wird,
**dadurch gekennzeichnet, dass**
der Zusatzstoff-Port (29) ein nadelloser Zusatzstoff-Port (29) ist, der zur Aufnahme eines nadellosen männlichen Konnektors einer nadellosen Zusatzstoff-Transfervorrichtung angeordnet ist und eine freiliegende nadellose Zugangsfläche (41) mit einem vorgeformten Schlitz zur Aufnahme des nadellosen männlichen Konnektors dergestalt einschließt, dass das selbstdichtende Zugangsventil (32) nach abdichtender Einbringung des nadellosen männlichen Konnektors darin aus dem normalerweise geschlossenen Zustand in den offenen Zustand gebracht wird, wobei die freiliegende nadellose Zugangsfläche (41) mit einem distalen Rand (36) des Zusatzstoff-Ports (29) bündig abschließt, wobei die freiliegende nadellose Zugangsfläche (41) für mehrfache sterile abdichtende Einbringungen des nadellosen männlichen Konnektors durch diese sterilisiert werden kann,
und der nadellose Zusatzstoff-Port (29) sich in direkter und kontinuierlicher Fluidkommunikation mit dem Ersatz-iv-Port (43) dergestalt befindet, dass sich der iv-Einstechdorn, der normalerweise geschlossene nadellose Zusatzstoff-Port und der Ersatz-iv-Port in einer direkten und kontinuierlichen Dreiwege-Fluidkommunikation befinden, wobei der flüssige Zusatzstoff der Zusatzstoff-Transfervorrichtung als Alternative direkt an einen Patienten verabreicht werden kann.

2. Ventil nach Anspruch 1, wobei ein männlicher Konnektor mittels eines Schraubgewindes an dem nadellosen Zusatzstoff-Port befestigt ist.

3. Ventil nach Anspruch 1 oder 2, wobei der iv-Einstechdorn (22) und der nadellose Zusatzstoff-Port (29) eine spritzgegossene monolithische Kunststoffstruktur bilden.

## Revendications

1. Valve de contrôle d'additif (13) destinée à être utilisée avec un récipient de fluide (10) contenant un liquide de perfusion et possédant un port intraveineux (IV) (11) pour l'administration du liquide de perfusion, et un dispositif de transfert d'additif (14) contenant un additif liquide et possédant un raccord mâle (16) pour l'administration de l'additif liquide,
la valve de contrôle d'additif (13) comprenant un corps de raccord à trois branches (21) pourvu d'une pointe IV (22) destinée à être introduite de manière étanche dans le port IV (11), d'un port IV de substitution (43) destiné à recevoir de manière étanche une pointe IV, et d'un port d'additif normalement fermé (29) pourvu d'un raccord femelle destiné à recevoir de manière étanche le raccord mâle (16) du dispositif de transfert d'additif,
ladite pointe IV (22) étant en liaison fluidique directe et continue avec ledit port IV de substitution (43), et ledit port d'additif normalement fermé (29) étant en liaison fluidique directe et continue avec ladite pointe IV afin de permettre ainsi à l'additif liquide du dispositif de transfert d'additif de se mélanger avec le liquide de perfusion,
ledit port d'additif (29) comportant une valve d'accès auto-étanchéifiante (32) qui est forcée à passer d'une condition normalement fermée à une condition ouverte lorsqu'un raccord mâle y est introduit de manière étanche,
**caractérisée en ce que**
ledit port d'additif (29) est un port d'additif sans aiguille (29) adapté pour recevoir un raccord mâle sans aiguille d'un dispositif de transfert d'additif sans aiguille et comporte une surface d'accès sans aiguille exposée (41) possédant une fente préformée destinée à recevoir le raccord mâle sans aiguille de façon à forcer la valve d'accès auto-étanchéifiante (32) à passer de la condition normalement fermée à la condition ouverte lorsque le raccord mâle sans aiguille y est introduit de manière étanche, la surface d'accès sans aiguille exposée (41) affleurant un rebord distal (36) du port d'additif (29), la surface d'accès sans aiguille exposée (41) pouvant ainsi être stérilisée afin de pouvoir introduire le raccord mâle sans aiguille à travers elle de manière étanche et stérile à de multiples reprises,
et **en ce que** ledit port d'additif sans aiguille (29) est en liaison fluidique directe et continue avec ledit port IV de substitution (43) de telle façon que ladite pointe IV, ledit port d'additif sans aiguille normalement fermé et ledit port IV de substitution sont en liaison fluidique directe et continue à trois voies, l'additif liquide du dispositif de transfert d'additif pouvant ainsi être administré directement en alternance à un patient.

2. Valve selon la revendication 1, dans laquelle un raccord mâle est monté au moyen d'un filetage sur ledit port d'additif sans aiguille.

3. Valve selon la revendication 1 ou la revendication 2, dans laquelle ladite pointe IV (22) et ledit port d'additif sans aiguille (29) sont une structure monobloc en matière plastique moulée par injection.
